# EUROPEAN PATENT APPLICATION

(11) **EP 4 834 385 A1**
(43) Date of publication of application: **04.11.2026**
(21) Application number: 24911986.8
(22) Date of filing: 28.10.2024
(51) Int. Cl.: A61M 1/16

(54) **HOLDER DEVICE AND ATTACHMENT METHOD**

(30) Priority: 27.12.2023 JP 2023221894
(71) Applicant: Nikkiso Co., Ltd., Tokyo 150-6022 (JP)
(72) Inventor: MOCHIZUKI, Hiroaki, Higashimurayama-shi Tokyo 189-8520 (JP)
(74) Representative: Algemeen Octrooi- en Merkenbureau B.V.
(86) International application number: PCT/JP2024/038299
(87) International publication number: WO 2025/142093

(57) **Abstract**

A holder apparatus (10) includes: a support part that supports a filter (70) extending in an axial direction, regulates displacement of the filter (70) in the axial direction, and permits rotation of the filter (70) around the axial direction; a first receiving part (24) that receives a first side port (84) extending from a side (78) of the filter (70) in a radial direction and regulates displacement of the first side port (84) in the radial direction, when the filter (70) supported by the support part is rotated in a predetermined direction around the axial direction; and a first side connection port (34) that is displaced in a direction approaching the first side port (84) placed in the first receiving part (24) and that is adapted to be connected to the first side port (84).

## Description

### TECHNICAL FIELD

The present invention relates to a filter holder apparatus and a mounting method.

### BACKGROUND ART

A dialysis machine includes a holder apparatus for holding a filter detachably. A filter is, for example, a blood purifier connected to a blood circuit or a filter for purifying a dialysate supplied to the blood purifier. The holder apparatus includes multiple connection ports for connecting, to each of multiple ports of the filter, a corresponding tube. For example, the holder apparatus includes a connection mechanism that enables connection between the multiple ports and the multiple connection ports with a lever operation (see, for example, patent literature 1).

### RELATED-ART LITERATURE

### PATENT LITERATURE

[Patent Literature 1] JP2015-177973

### SUMMARY OF INVENTION

### TECHNICAL PROBLEM

To connect a connection port to a port of the filter, it is necessary to apply a force in the direction of extension of the ports. Applying a force to the filter for connection can cause a misalignment of the filter. If the misalignment is large, connection may fail. An attempt to fix the filter firmly to prevent a misalignment of the filter connected will require more effort to mount the filter.

The present invention addresses the issue described above, and an illustrative purpose thereof is to provide a technology to improve the workability of mounting a filter on a holder apparatus.

### SOLUTION TO PROBLEM

A holder apparatus according to an embodiment of the present invention includes: a support part that supports a filter extending in an axial direction, regulates displacement of the filter in the axial direction, and permits rotation of the filter around the axial direction; a receiving part that receives a side port extending from a side of the filter in a radial direction and regulates displacement of the side port in the radial direction, when the filter supported by the support part is rotated in a predetermined direction around the axial direction; and a side connection port that is displaced in a direction approaching the side port placed in the receiving part and that is adapted to be connected to the side port.

Another embodiment of the present invention relates to a mounting method. The method includes: causing a filter extending in an axial direction to be supported by a support part of a holder apparatus; rotating the filter supported by the support part in a predetermined direction around the axial direction and inserting a side port extending from a side of the filter in a radial direction into a receiving part of the holder apparatus; and connecting a side connection port of the holder apparatus to the side port placed in the receiving part.

### ADVANTAGEOUS EFFECTS OF INVENTION

According to the present invention, the workability of mounting a filter on a holder apparatus can be improved.

### BRIEF DESCRIPTION OF DRAWINGS

[Fig. 1] Fig. 1 is a side view schematically showing a configuration of a holder apparatus according to the first embodiment.
[Fig. 2] Fig. 2 is a front view schematically showing a configuration of the holder apparatus according to the first embodiment.
[Fig. 3] Fig. 3 is a cross-sectional view schematically showing an internal structure of the filter.
[Fig. 4] Fig. 4 is a side view schematically showing a configuration of the holder apparatus with the filter being removed.
[Fig. 5] Fig. 5 is a front view schematically showing a configuration of the holder apparatus with the filter being removed.
[Fig. 6] Fig. 6 schematically shows a structure of the first support part.
[Fig. 7] Fig. 7 schematically shows a structure of the first support part.
[Fig. 8] Fig. 8 schematically shows a structure of the first receiving part.
[Fig. 9] Fig. 9 schematically shows a structure of the first receiving part.
[Fig. 10] Fig. 10 schematically shows a structure of the rotation suppression part.
[Fig. 11] Fig. 11 is a flowchart showing a flow of a mounting method according to the embodiment.
[Fig. 12] Fig. 12 schematically shows a method of mounting the filter on the holder apparatus.
[Fig. 13] Fig. 13 schematically shows a structure of the first receiving part according to a variation.
[Fig. 14] Fig. 14 schematically shows a structure of a first receiving part according to another variation.
[Fig. 15] Fig. 15 is a side view schematically showing a configuration of a holder apparatus according to the second embodiment.
[Fig. 16] Fig. 16 is a front view schematically showing a configuration of the holder apparatus according to the second embodiment.

### DESCRIPTION OF EMBODIMENTS

A description will be given of an embodiment of the present invention with reference to the drawings. The numerals are used in the description to denote the same elements and a duplicate description is omitted as appropriate. To facilitate the understanding, the relative dimensions of the constituting elements in the drawings do not necessarily mirror the actual relative dimensions.

### (First embodiment)

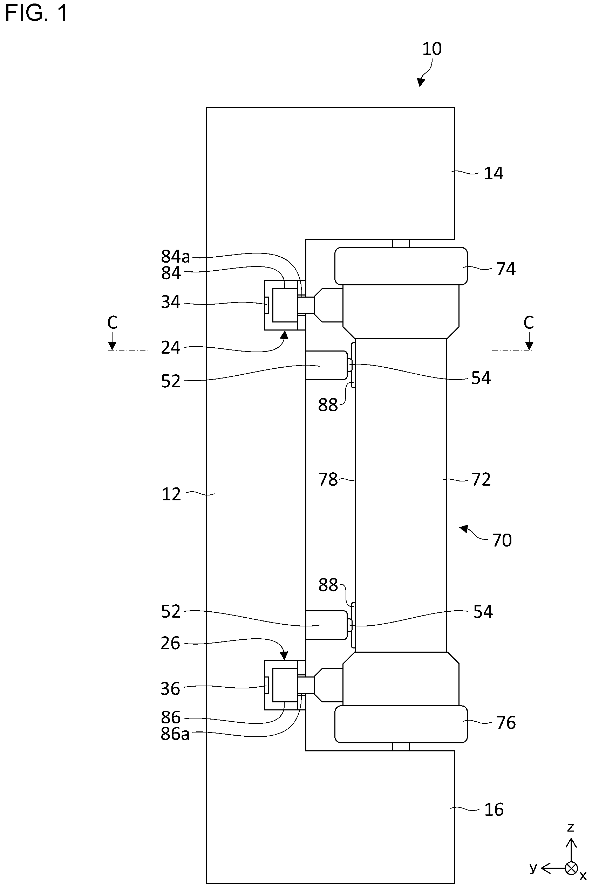

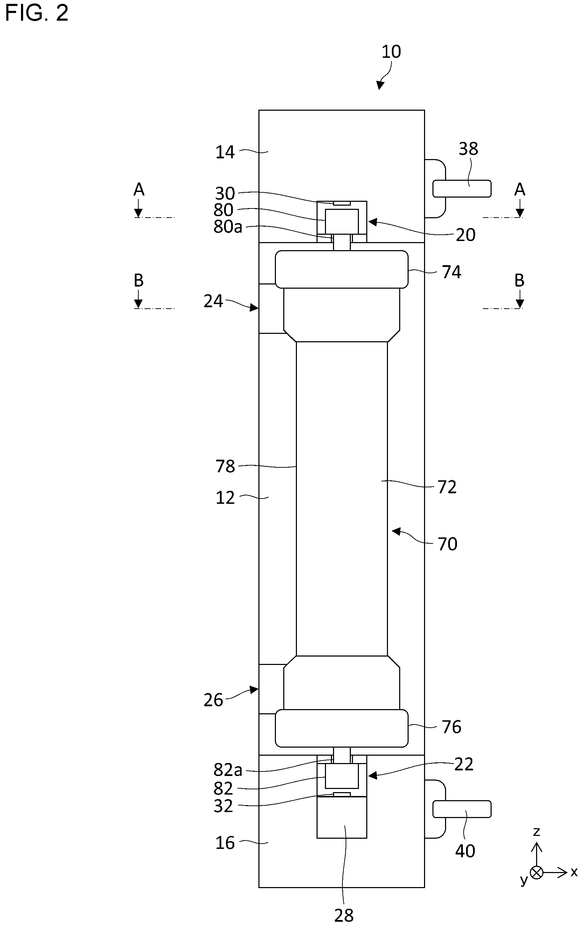

Fig. 1 is a side view schematically showing a configuration of a holder apparatus 10 according to the first embodiment. Fig. 2 is a front view schematically showing a configuration of the holder apparatus 10 according to the first embodiment. The holder apparatus 10 is an apparatus for holding a filter 70 and connecting to the filter 70. The filter 70 is, for example, a particulate filter for filtration and purification of a dialysate used in dialytic therapy. The holder apparatus 10 is, for example, provided in a dialysis monitoring apparatus to monitor dialytic therapy.

The filter 70 includes a housing 72 extending in the axial direction in a cylindrical shape, and a filtering member such as a hollow fiber is provided inside the housing 72. The filter 70 includes a first end port 80, a second end port 82, a first side port 84, and a second side port 86, as ports that serve as inlets or outlets for the liquid flowing inside the filter 70.

The first end port 80 extends from a first end 74 of the housing 72 in the axial direction. The second end port 82 extends in the axial direction from a second end 76 of the housing 72 opposite to the first end 74. The first side port 84 and the second side port 86 extend from a side 78 of the housing 72 in the radial direction. The first side port 84 is provided in the vicinity of the first end 74. The second side port 86 is provided in the vicinity of the second end 76.

The filter 70 can include ribs 88 protruding from the side 78 of the housing 72 in the radial direction. The ribs 88 are provided, for example, between the first side port 84 and the second side port 86. The ribs 88 can be provided such that the positions thereof in the circumferential direction as viewed from the central axis of the filter 70 coincide with the first side port 84 and the second side port 86.

In this specification, the direction in which the filter 70 extends may be referred to as the axial direction, the direction orthogonal to the axial direction may be referred to as the radial direction, and the direction around the axial direction may be referred to as the circumferential direction. The drawings show coordinate axes in which the axial direction is defined as the z direction to help understand the embodiment. The x direction and the y direction, which are orthogonal to the z direction, are one of radial directions. The direction in which the first side port 84 and the second side port 86 extend from the filter 70 connected to the holder apparatus 10 is defined as the +y direction.

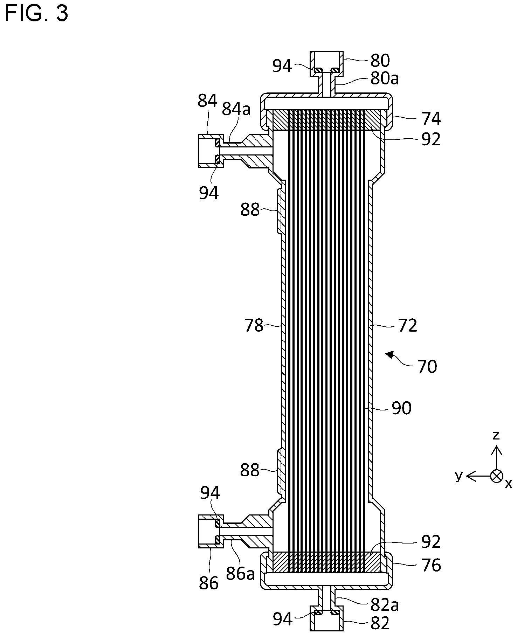

Fig. 3 is a cross-sectional view schematically showing an internal structure of the filter 70. The filter 70 has a hollow fiber 90 and a sealing member 92 that are placed inside the housing 72. The hollow fiber 90 extends from the first end 74 to the second end 76 of the housing 72 in the axial direction. The sealing member 92 is provided at the first end 74 and the second end 76 of housing 72, respectively, to fix the ends of the hollow fiber 90.

The first end port 80 and the second end port 82 communicate with a flow path inside the hollow fiber 90. For example, a liquid flowing in from the second end port 82 and passing inside the hollow fiber 90 can flow out of the first end port 80. The first side port 84 and the second side port 86 communicate with a flow path outside the hollow fiber 90. For example, a liquid flowing in from the second end port 82 and passing inside the hollow fiber 90 can flow out of the first side port 84 or the second side port 86. Each of the ports 80, 82, 84, and 86 of the filter 70 may be either on the inflow side or the outflow side and can be configured as appropriate depending on how the filter 70 is used.

The ports 80, 82, 84 and 86 have small-diameter parts 80a, 82a, 84a, and 86a, respectively. The small-diameter parts 80a, 82a, 84a, and 86a are used to engage with the holder apparatus 10 and fix the filter 70 to the holder apparatus 10. A gasket 94 is inserted in each of the ports 80, 82, 84, and 86.

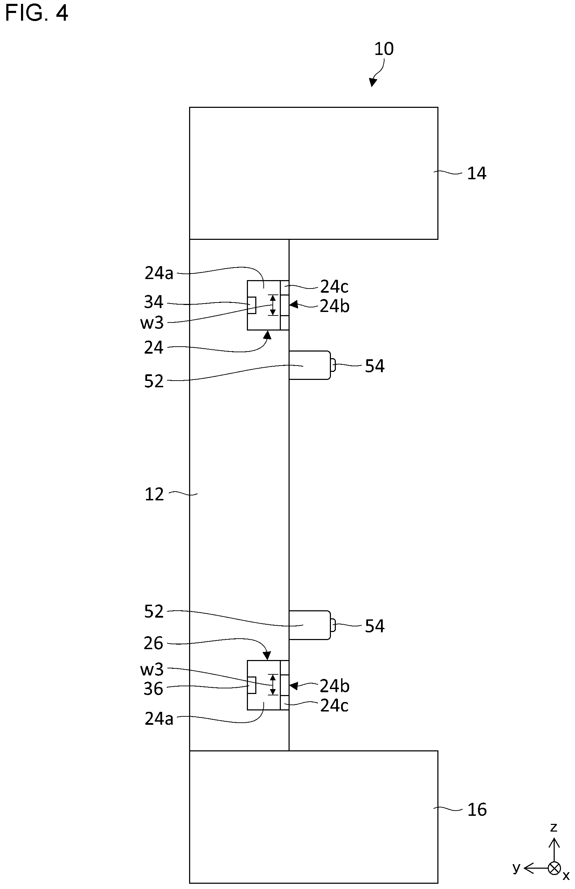

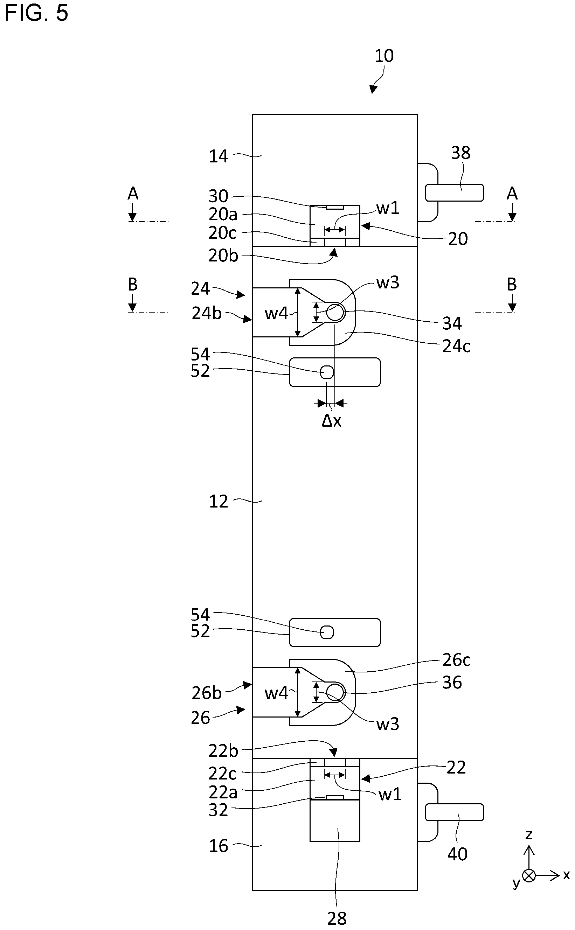

Fig. 4 is a side view schematically showing a configuration of the holder apparatus 10 and shows a state in which the filter 70 in Fig. 1 is removed. Fig. 5 is a front view schematically showing a configuration of the holder apparatus 10 and shows a state in which the filter 70 in Fig. 2 is removed.

The holder apparatus 10 includes a main body 12, a first arm 14, and a second arm 16. The main body 12 is a part that extends in parallel with the filter 70 in the axial direction. The first arm 14 and the second arm 16 are parts that extend from the main body 12 in the radial direction. For example, the first arm 14 extends from vertically above the main body 12 in the radial direction, and the second arm 16 extends from vertically below the main body 12 in the radial direction. The first arm 14 and the second arm 16 are separated in the axial direction, and the filter 70 is placed between the first arm 14 and the second arm 16.

The holder apparatus 10 further includes a first support part 20, a second support part 22, a first receiving part 24, a second receiving part 26, a first end connection port 30, a second end connection port 32, a first side connection port 34, a second side connection port 36, a first control lever 38, and a second control lever 40.

The first support part 20 is provided in the first arm 14. The first support part 20 receives the first end port 80 of the filter 70 and supports the first end port 80. The first support part 20 regulates the displacement of the first end port 80 in the axial direction and regulates the displacement of the first end port 80 in the x direction. The first support part 20 permits the displacement of the first end port 80 in the y direction and permits the rotation thereof around the axial direction.

The first support part 20 has a recess 20a that houses the first end port 80. The first support part 20 has an engagement part 20c in which a notch 20b that engages with the small-diameter part 80a of the first end port 80 is formed. The engagement part 20c has a thickness in the axial direction. The engagement part 20c is inserted between the first end 74 and the first end port 80 of the filter 70 and regulates the displacement of the first end port 80 in the axial direction and the x direction.

The first end connection port 30 is provided in the first support part 20. The first end connection port 30 extends in the axial direction and is displaceable in the axial direction. The leading end of the first end connection port 30 is located in the recess 20a of the first support part 20. The first end connection port 30 is displaced in the direction approaching the first end port 80 placed in the recess 20a of the first support part 20 and is inserted into the first end port 80. The first end connection port 30 is configured to maintain, by a biasing means such as a spring, a connected state in which the first end connection port 30 is inserted in the first end port 80. The first end connection port 30 in the connected state abuts the gasket 94 inside the first end port 80 and is connected to the first end port 80 in a watertight manner. The first end connection port 30 is configured to be displaced in the direction away from the first end port 80 and to maintain a released state separated from the first end port 80. The first end connection port 30 is configured to be switched between the connected state and the released state by manipulation of the first control lever 38. The first control lever 38 can be, for example, provided on the side of the first arm 14.

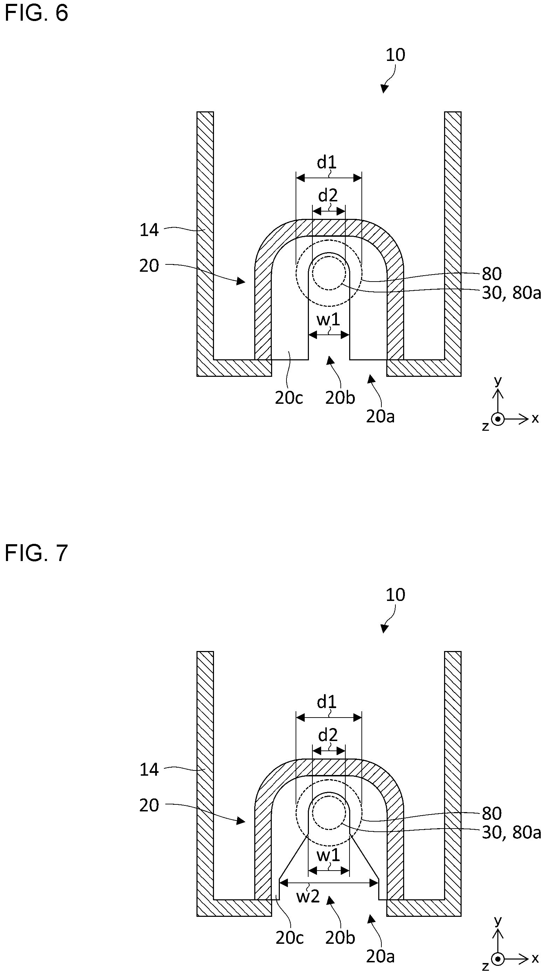

Fig. 6 schematically shows a structure of the first support part 20 and corresponds to an A-A cross-section of Fig. 2 or Fig. 5. Fig. 6 shows the position of the first end connection port 30 and the positions of the first end port 80 and the small-diameter part 80a at connection positions facing the first end connection port 30 by dashed lines. The notch 20b extends in the y direction toward the connection position corresponding to the first end connection port 30 and is configured to have a constant width w1 in the x direction. The width w1 of the notch 20b in the x-direction is smaller than the diameter d1 of the first end port 80 and larger than the diameter d2 of the small-diameter part 80a. The first end port 80 can be placed at or guided to the connection position where it can be connected to the first end connection port 30 by inserting the first end port 80 into the notch 20b and moving it to the notch end.

Fig. 7 schematically shows a structure of the first support part 20 and shows a variation in which the width of the notch 20b in the x-direction is not constant. In the example of Fig. 7, the width w2 of the notch 20b in the x direction near the entrance is relatively large, and the width in the x direction grows smaller toward the connection position corresponding to the first end connection port 30 (i.e., toward the notch end) until the width is w1. The width w2 of the notch 20b near the entrance is larger than the diameter d1 of the first end port 80. Configuring the width w2 of the notch 20b near the entrance to be relatively large makes it easier to insert the first end port 80 into the first support part 20. Further, configuring the width w1 of the notch 20b near the connection position to be relatively small makes it possible to guide the first end port 80 to the connection position corresponding to the first end connection port 30 properly.

Referring back to Fig. 4 or Fig. 5, the second support part 22 is provided in the second arm 16. The second support part 22 receives the second end port 82 of the filter 70 and supports the second end port 82. The second support part 22 regulates the displacement of the second end port 82 in the axial direction and regulates the displacement of the second end port 82 in the x direction. The second support part 22 permits the displacement of the second end port 82 in the y direction and permits the rotation thereof around the axial direction.

The second support part 22 can be configured similarly to the first support part 20. The second support part 22 has a recess 22a that houses the second end port 82. The second support part 22 has an engagement part 22c in which a notch 22b that engages with the small-diameter part 82a of the second end port 82 is formed. The engagement part 22c has a thickness in the axial direction. The engagement part 22c is inserted between the second end 76 and the second end port 82 of the filter 70 and regulates the displacement of the second end port 82 in the axial direction. The width w of the notch 22b in the x direction is smaller than the diameter of the second end port 82 and larger than the diameter of the small-diameter part 82a.

The second end connection port 32 is provided in the second support part 22. The second end connection port 32 extends in the axial direction and is displaceable in the axial direction. The leading end of the second end connection port 32 is located in the recess 22a of the second support part 22. The second end connection port 32 is displaced in the direction approaching the second end port 82 placed in the second support part 22 and is inserted into the second end port 82. Like the first end connection port 30, the second end connection port 32 is configured to maintain, by a biasing means such as a spring, a connected state in which the second end connection port 32 is inserted in the second end port 82. The second end connection port 32 abuts the gasket 94 inside the second end port 82 and is connected to the second end port 82 in a watertight manner. The second end connection port 32 is configured to be displaced in the direction away from the second end port 82 and to maintain a released state separated from the second end port 82. The second end connection port 32 is configured to be switched between the connected state and the released state by manipulation of the second control lever 40. The second control lever 40 can be, for example, provided on the side of the second arm 16.

A drain port 28 can be provided in the lower part of the second support part 22. The drain port 28 has, for example, an inclined surface and is configured to discharge the liquid accumulating in the recess 22a of the second support part 22 outside. By providing the drain port 28, the liquid flowing from the second end port 82 can be prevented from accumulating in the second support part 22 as the second end port 82 and the second end connection port 32 are disconnected in the second support part 22.

The first support part 20 and the second support part 22 are configured to cooperate to position the central axis of the filter 70. The filter 70 supported by the first support part 20 and the second support part 22 can be rotated around the central axis in the circumferential direction.

The first receiving part 24 is provided in the main body 12. The first receiving part 24 receives the first side port 84 of the filter 70 and holds the first side port 84. The first receiving part 24 regulates the displacement of the first side port 84 in the axial direction and regulates the displacement of the first side port 84 in the radial direction. The first receiving part 24 regulates a range of rotation of the first side port 84 around the axial direction.

The first receiving part 24 has a recess 24a that houses the first side port 84. The first receiving part 24 has an engagement part 24c in which a notch 24b that engages with the small-diameter part 84a of the first side port 84 is formed. The engagement part 24c has a thickness in the y direction or the radial direction. The engagement part 24c is inserted between the side 78 of the filter 70 and the first side port 84 and regulates the displacement of the first side port 84 in the radial direction. The width w3 of the notch 24b in the z direction is smaller than the diameter of the first side port 84 and larger than the diameter of the small-diameter part 84a.

The first side connection port 34 is provided in the first receiving part 24. The first side connection port 34 extends in the radial direction or the y direction and is displaceable in the radial direction or the y direction. The leading end of the first side connection port 34 is located in the recess 24a of the first receiving part 24. The first side connection port 34 is displaced in the direction approaching the first side port 84 placed in the first receiving part 24 and is inserted into the first side port 84. Like the first end connection port 30, the first side connection port 34 is configured to maintain, by a biasing means such as a spring, a connected state in which the first side connection port 34 is inserted in the first side port 84. The first side connection port 34 abuts the gasket 94 inside the first side port 84 and is connected to the first side port 84 in a watertight manner. The first side connection port 34 is configured to be displaced in the direction away from the first side port 84 and to maintain a released state separated from the first side port 84. The first side connection port 34 is configured to be switched between the connected state and the released state by manipulation of the first control lever 38. The first control lever 38 is, for example, configured to switch the state of the first end connection port 30 and the first side connection port 34 via a link mechanism.

The notch 24b of the first receiving part 24 extends toward the connection position corresponding to the first side connection port 34 in the x direction. The notch 24b is configured in such a way that the width w4 of the notch 24b in the axial direction near the entrance is relatively large, and the width w3 in the axial direction grows smaller toward the connection position corresponding to the first side connection port 34. Configuring the width w4 of the notch 24b near the entrance to be relatively large makes it easier to insert the first side port 84 into the first receiving part 24. Further, configuring the width w3 of the notch 24b near the connection position to be relatively small makes it possible to guide the first side port 84 to the connection position corresponding to the first side connection port 34 properly.

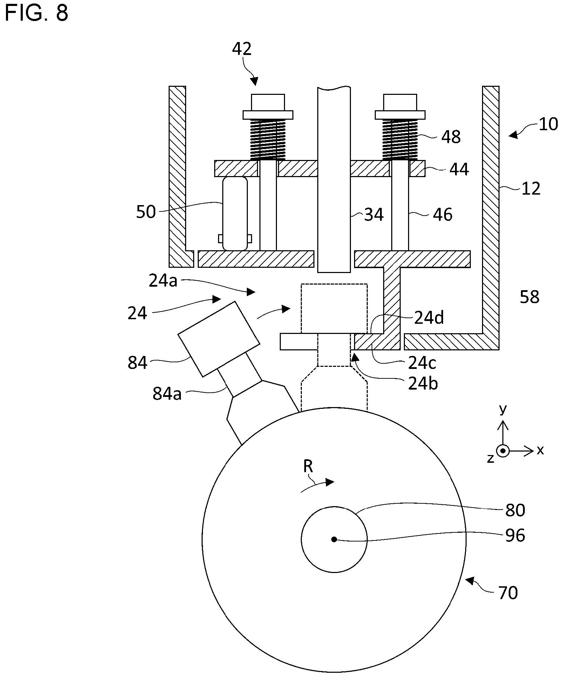

Fig. 8 schematically shows a structure of the first receiving part and corresponds to a B-B cross-section of Fig. 2 or Fig. 5. The filter 70 shown in Fig. 8 is supported by the first support part 20 and the second support part 22, and the first side port 84 is placed at a position misaligned from the first receiving part 24 in the circumferential direction. The filter 70 is rotatable around the central axis 96. When the filter 70 is rotated in a predetermined mounting direction (e.g., clockwise) indicated by the arrow R, the first side port 84 can be placed in the first receiving part 24 as indicated by the dashed line. The small-diameter part 84a of the first side port 84 placed in the first receiving part 24 is placed inside the notch 24b. The first side port 84 placed in the first receiving part 24 is in contact with an inner surface 24d of the engagement part 24c, and the displacement of the first side port 84 in the radial direction or the y direction is regulated by the engagement part 24c. The first side port 84 placed in the first receiving part 24 cannot rotate further in the predetermined mounting direction (e.g., clockwise) indicated by the arrow R. As a result, the first side port 84 is guided to the connection position facing the first side connection port 34.

Fig. 8 further shows a structure of a switching part 42 for switching between the connected state and the released state of the first side connection port 34. Fig. 8 shows the switching part 42 in the released state. The switching part 42 includes a base 44, a guide 46, a spring 48, and a cam 50. The base 44 is a plate-like member on which the first side connection port 34 is mounted. The guide 46 is a rod-shaped member extending from the first receiving part 24 in the radial direction or the y direction. The base 44 is displaceable along the guide 46 in the radial direction or the y direction. The spring 48 is provided on the outer circumference of the guide 46 and biases the base 44 toward the first receiving part 24 in the radial direction or the y direction. The cam 50 is placed between the first receiving part 24 and the base 44 and switches the distance between the first receiving part 24 and the base 44.

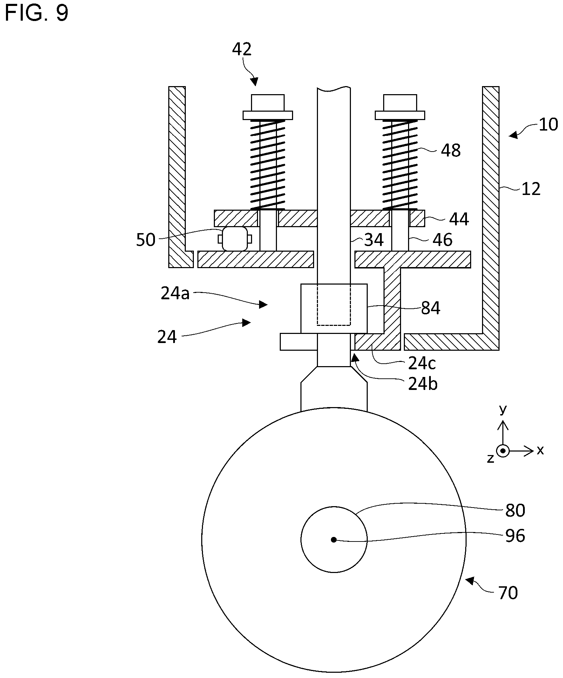

Fig. 9 shows the switching part 42 in the connected state. When the cam 50 is rotated to reduce the distance between the first receiving part 24 and the base 44, the base 44 is biased by the spring 48 to insert the first side connection port 34 into the first side port 84. When the cam 50 is rotated in the connected state, the released state of Fig. 8 can be resumed. The cam 50 is mechanically connected to the first control lever 38, and the cam 50 can be rotated by manipulating the first control lever 38.

Referring back to Fig. 4 or Fig. 5, the second receiving part 26 is provided in the main body 12. The second receiving part 26 receives the second side port 86 of the filter 70 and holds the second side port 86. The second receiving part 26 can be configured similarly to the first receiving part 24. The second receiving part 26 regulates the displacement of the second side port 86 in the axial direction and regulates the displacement of the second side port 86 in the radial direction. The second receiving part 26 regulates a range of rotation of the second side port 86 around the axial direction.

The second receiving part 26 has a recess 26a that houses the second side port 86. The second receiving part 26 has an engagement part 26c in which a notch 26b that engages with the small-diameter part 86a of the second side port 86 is formed. The engagement part 26c has a thickness in the y direction or the radial direction. The engagement part 26c is inserted between the side 78 of the filter 70 and the second side port 86 and regulates the displacement of the second side port 86 in the radial direction. The width w3 of the notch 26b in the z direction is smaller than the diameter of the second side port 86 and larger than the diameter of the small-diameter part 86a.

The second side connection port 36 is provided in the second receiving part 26. The second side connection port 34 extends in the radial direction or the y direction and is displaceable in the radial direction or the y direction. The leading end of the second side connection port 36 is located in the recess 26a of the second receiving part 26. The second side connection port 36 is displaced in the direction approaching the second side port 86 placed in the second receiving part 26 and is inserted into the second side port 86. Like the first side connection port 34, the second side connection port 36 is configured to maintain, by a biasing means such as a spring, a connected state in which the second side connection port 36 is inserted in the second side port 86. The second side connection port 36 abuts the gasket 94 inside the second side port 86 and is connected to the second side port 86 in a watertight manner. The second side connection port 36 is configured to be displaced in the direction away from the second side port 86 and to maintain a released state separated from the second side port 86. The second side connection port 36 is, for example, configured to be switched between the connected state and the released state by manipulation of the second control lever 40. The second control lever 40 is, for example, configured to switch the state of the second end connection port 32 and the second side connection port 36 via a link mechanism.

As in the case of the first receiving part 24, the notch 26b of the second receiving part 26 extends in the x direction toward the connection position corresponding to the second side connection port 36. The notch 26b is configured in such a way that the width w4 of the notch 26b in the axial direction near the entrance is relatively large, and the width w3 in the axial direction grows smaller toward the connection position corresponding to the second side connection port 36. Configuring the width w4 of the notch 26b near the entrance to be relatively large makes it easier to insert the second side port 86 into the second receiving part 26. Further, configuring the width w3 of the notch 26b near the connection position to be relatively small makes it possible to guide the second side port 86 to the connection position corresponding to the second side connection port 36 properly.

The holder apparatus 10 can further include a rotation suppression part 52. The rotation suppression part 52 is provided in the main body 12 and protrudes from the main body 12 in the radial direction or the y direction. The rotation suppression part 52 has a protrusion 54 that engages with a rib 88 of the filter 70. The protrusion 54 is provided at a position misaligned from the center of the first side connection port 34 in the x direction and is provided at a position misaligned by Δx in the direction toward the entrance of the notch 24b of the first receiving part 24. The protrusion 54 suppresses the rotation of the filter 70 by engaging with the rib 88 of the filter 70. The protrusion 54 does not suppress the rotation of the filter 70 completely and has a height that allows the rib 88 of the filter 70 to get over the protrusion 54. The protrusion 54 has a convex curved surface that allows the rib 88 of the filter 70 to get over the protrusion 54 easily.

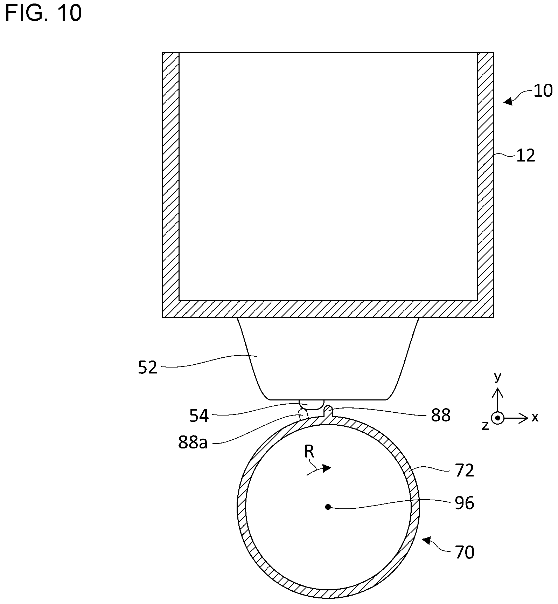

Fig. 10 schematically shows a structure of the rotation suppression part 52 and corresponds to a C-C cross-section of Fig. 1 or Fig. 4. Fig. 10 shows a state in which the first side port 84 of the filter 70 is placed at the connection position facing the first side connection port 34. A rib 88a indicated by the dashed line in Fig. 10 represents a state in which the port is not placed at the connection state. When the filter 70 is rotated in the predetermined mounting direction (e.g., clockwise) as indicated by the arrow R, the rib 88a can get over the protrusion 54. The rotation of the rib 88 that has got over the protrusion 54 in the opposite direction is suppressed by the protrusion 54. When a strong force is applied to the filter 70 to rotate it in the direction opposite to the arrow R, the rib 88 can get over the protrusion 54.

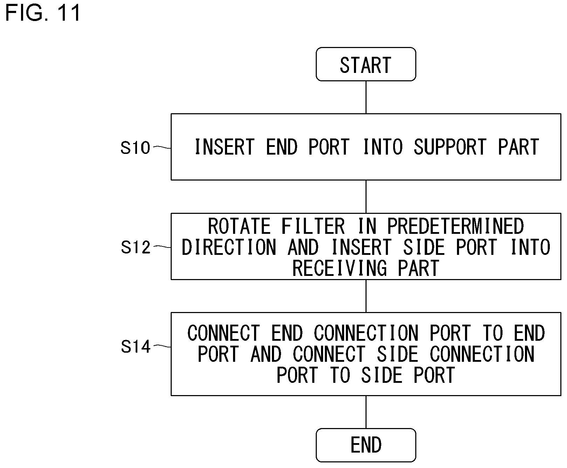

A description will now be given of a method to mount the filter 70 on the holder apparatus 10. Fig. 11 schematically shows a method of mounting the filter 70 on the holder apparatus 10 according to the embodiment. First, the filter 70 is inserted into the support part of the holder apparatus 10, and the support part of the holder apparatus 10 is caused to support the filter 70 (S10). For example, the filter 70 can be placed between the first support part 20 and the second support part 22 by inserting the first end port 80 into the first support part 20 and inserting the second end port 82 into the second support part 22.

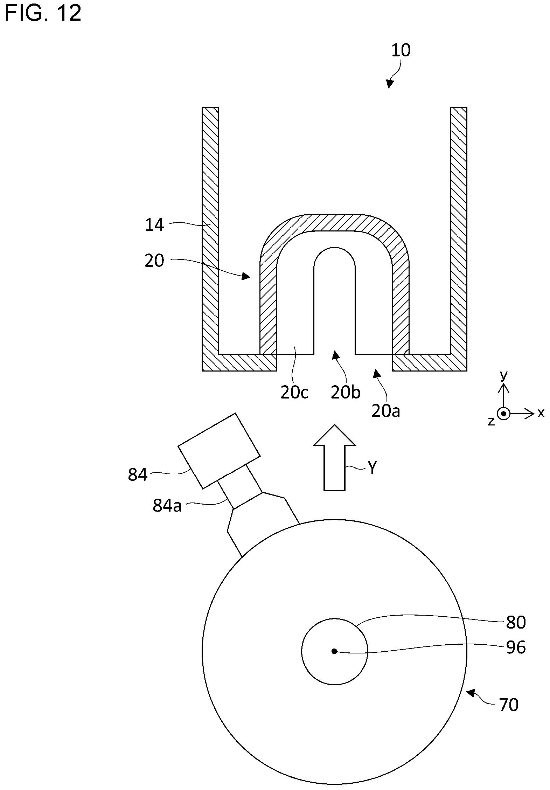

Fig. 12 schematically shows steps of inserting the first end port 80 into the first support part 20 of the holder apparatus 10. As indicated by the arrow Y of Fig. 12, the small-diameter part 80a of the first end port 80 can be inserted into the notch 20b of the first support part 20 by moving the filter 70 in the +y direction. In this process, the filter 70 is mounted in such a way that the first side port 84 extends in a direction different from the +y direction. This can prevent the first side port 84 from interfering with the first receiving part 24 when the first end port 80 is inserted in the first support part 20.

Referring back to Fig. 11, the filter 70 supported by the first support part 20 and the second support part 22 is then rotated in the predetermined mounting direction, and the side port of the filter 70 is inserted into the receiving part of the holder apparatus 10 (S12). As shown in Fig. 8, for example, the first side port 84 can be inserted into the first receiving part 24, and the second side port 86 can be inserted into the second receiving part 26, by rotating the filter 70 in the mounting direction indicated by the arrow R. In this process, the rib 88 of the filter 70 engages with the protrusion 54 of the rotation suppression part 52, thereby suppressing further rotation of the filter 70.

Then, the end connection port of the holder apparatus 10 is connected to the end port of the filter 70, and the side connection port of the holder apparatus 10 is connected to the side port of filter 70. For example, the first end connection port 30 can be connected to the first end port 80, and the first side connection port 34 can be connected to the first side port 84, by manipulating the first control lever 38 of the holder apparatus 10. Similarly, the second end connection port 32 can be connected to the second end port 82, and the second side connection port 36 can be connected to the second side port 86, by manipulating the second control lever 40 of the holder apparatus 10. Each of the connection ports 30, 32, 34, and 36 maintains a connected state in which it is biased toward the corresponding port by means of, for example, a mechanism similar to the switching part 42 shown in Fig. 9.

To remove the filter 70 from the holder apparatus 10, steps reverse to those of Fig. 11 may be employed. First, the ports 80, 82, 84, and 86 can be separated from the corresponding connection ports 30, 32, 34, and 36, respectively, by manipulating the first control lever 38 and the second control lever 40. Then, the first side port 84 can be taken out from the first receiving part 24, and the second side port 86 can be taken out from the second receiving part 26, by rotating the filter 70 in the direction opposite to the mounting direction. Finally, the filter 70 can be removed from the holder apparatus 10 by pulling the filter 70 out toward the front or in the -y direction.

According to the embodiment, the filter 70 can be mounted on the holder apparatus 10 in three steps including: inserting the filter 70 toward the holder apparatus 10, rotating the filter 70, and manipulating the control lever so that the workability of attaching and detaching the filter 70 can be improved. According to the embodiment, the side port is inserted into the receiving part of the holder apparatus 10 by rotating the filter 70 so that the displacement of the filter 70 in the radial direction can be regulated by the receiving part. Therefore, the displacement of the filter 70 can be prevented, even if a force is applied to the filter 70 both in the axial direction and the radial direction when the connection ports are connected to the respective ports of the filter 70. As a result, connection failure due to misalignment at the time of connecting the filter 70 can be prevented, and the workability of attaching and detaching the filter 70 can be improved.

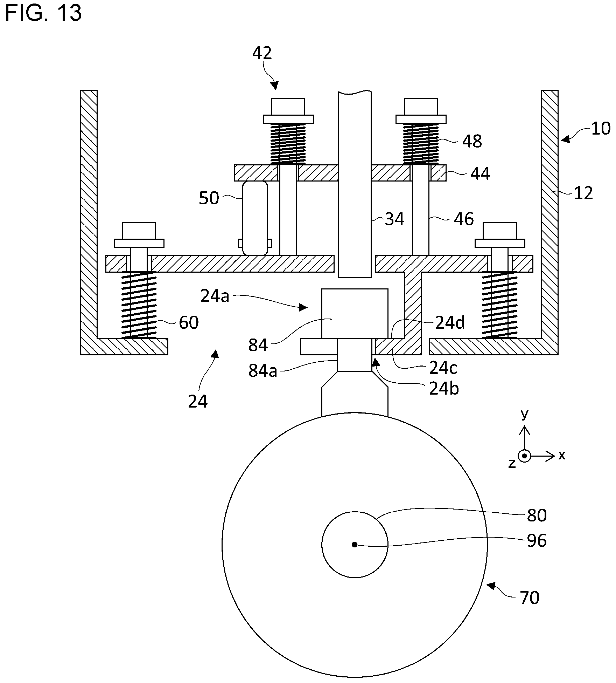

Fig. 13 schematically shows a structure of the first receiving part 24 according to a variation and corresponds to Fig. 8 above. The first receiving part 24 shown in Fig. 13 is mounted on the main body 12 via a spring 60. The spring 60 biases the first receiving part 24 toward the main body 12 in the +y direction. The spring 60 applies a force in the direction of pulling the first side port 84 inserted in the first receiving part 24 in the +y direction. This allows the first side port 84 and the inner surface 24d of the first receiving part to be in firmer contact, regulating the displacement of the first side port 84. Therefore, the spring 60 can function as a rotation suppression part that suppresses the rotation of the first side port 84 placed at the connection position facing the first side connection port 34.

The first receiving part 24 is mounted on the main body 12 via the spring 60 extending in the y direction and so can be slightly displaced relative to the main body 12 in the x and z directions. In other words, the first receiving part 24 is swingable with respect to the main body 12 in the x and z directions. As a result, even when the first side port 84 is placed slightly misaligned from the design position due to tolerance in manufacturing of the filter 70, etc., a slight swinging motion of the first receiving part 24 allows receiving the first side port 84 at the misaligned position.

A structure similar to the spring 60 may be added between the main body 12 and the second receiving part 26. A structure similar to the spring 60 may also be added between the first arm 14 and the first support part 20 or between the second arm 16 and the second support part 22. In this case, each of the first support part 20, the second support part 22, the first receiving part 24, and the second receiving part 26 is independently swingable with respect to the main body 12. This can absorb misalignment caused by the manufacturing tolerance of each of the first end port 80, the second end port 82, the first side port 84, and the second side port 86.

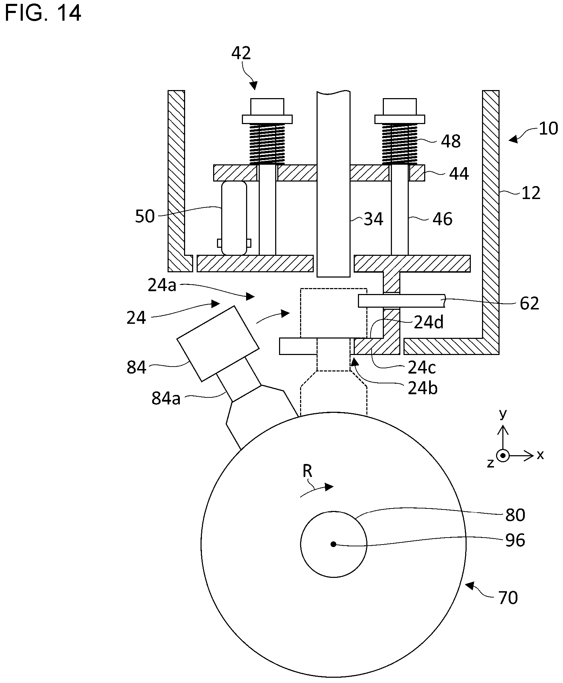

Fig. 14 schematically shows a structure of a first receiving part 24 according to another variation and corresponds to Fig. 8 above. The first receiving part 24 shown in Fig. 14 has a detection part 62 that detects that the first side port 84 is inserted in the first receiving part 24. The detection part 62 is a rod-shaped member extending in the x direction, and the leading end of the detection part 62 is located in the recess 24a of the first receiving part 24. When the first side port 84 is inserted in the first receiving part 24, the detection part 62 is displaced in the +x direction and detects the insertion of the first side port 84. The detection part 62 is, for example, mechanically connected to a switching inhibition mechanism or a locking mechanism that inhibits the rotation of the cam 50.

When the insertion of the first side port 84 is detected by the detection part 62, the switching part 42 may unlock the cam 50 and automatically switch from the released state to the connected state in response to the detection. In this case, the first side connection port 34 can be automatically connected to the first side port 84 merely by inserting the first side port 84 into the first receiving part 24 properly.

The switching part 42 may unlock the cam 50 and permit rotation when the insertion of the first side port 84 is detected by the detection part 62. In this case, the switching part 42 does not automatically switch the state in response to the detection by the detection part 62 but switches the state in response to the manipulation of the first control lever 38. The switching part 42 locks the cam 50 to inhibit rotation when the insertion of the first side port 84 is not detected by the detection part 62. In this case, the first control lever 38 cannot be manipulated, and state switching by the manipulation of the first control lever 38 is inhibited. According to this variation, the connection work by the control lever can be performed only when the first side port 84 is properly inserted in the first receiving part 24 so that connection failure is prevented.

The configuration of the switching part 42 and the detection part 62 according to the variation may be applied to the second receiving part 26.

### (Second embodiment)

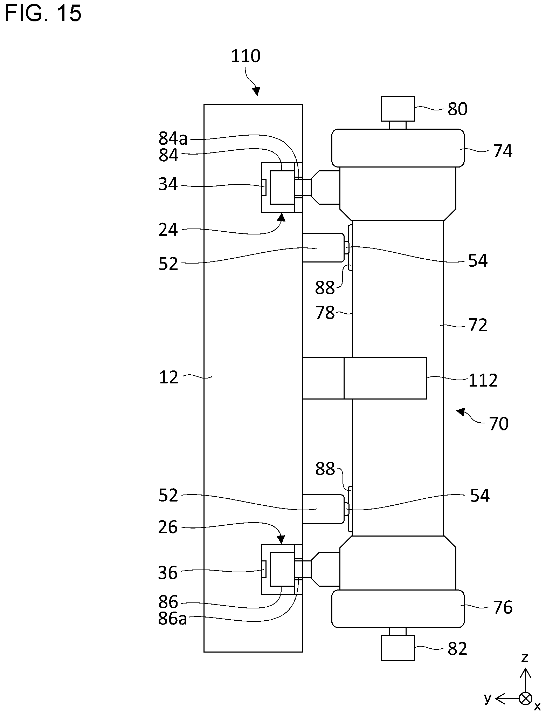

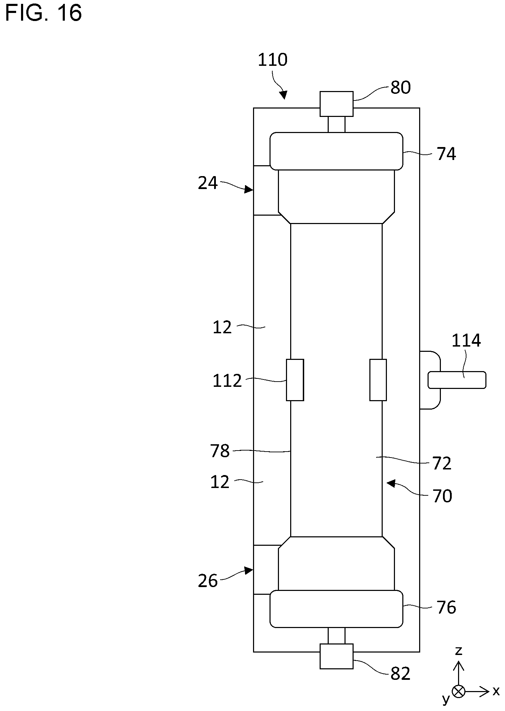

Fig. 15 is a side view schematically showing a configuration of a holder apparatus 110 according to the second embodiment. Fig. 16 is a front view schematically showing a configuration of the holder apparatus 110 according to the second embodiment. The holder apparatus 110 according to the second embodiment is different from the first embodiment described above in that the apparatus includes a support part 112 that supports the side 78 of the housing 72 of the filter 70, instead of supporting the first end port 80 and the second end port 82 of the filter 70. The following description of the holder apparatus 110 according to the second embodiment highlights differences from the first embodiment described above. A description of common features is omitted as appropriate.

The holder apparatus 110 includes the main body 12 similar to that of the first embodiment. The holder apparatus 110 does not include the first arm 14 and the second arm 16. The holder apparatus 110 further includes the support part 112, the first receiving part 24, the second receiving part 26, the first side connection port 34, the second side connection port 36, and a control lever 114. The first receiving part 24, the second receiving part 26, the first side connection port 34, and the second side connection port 36 can be configured similarly to the first embodiment described above.

The support part 112 extends from the main body 12 in the radial direction. The support part 112 is, for example, provided near the center of the main body 12 and is provided at a position between the first receiving part 24 and the second receiving part 26. The support part 112 grips and holds the housing 72 of the filter 70. The support part 112 regulates the displacement of the filter 70 in the axial direction and permits the rotation of the filter 70 around the axial direction. A plurality of support parts 112 may be provided at intervals in the axial direction.

The control lever 114 is provided on the side of the main body 12. The connected state and the released state of the first side connection port 34 and the second side connection port 36 can be switched by manipulating the control lever 114.

A description will now be given of a method of mounting the filter 70 on the holder apparatus 110. The mounting method is similar to that of the first embodiment, and the filter can be mounted in a flow similar to that of Fig. 11. First, the filter 70 is inserted into the support part 112. Then, the filter 70 is rotated in the predetermined mounting direction, and the side port is inserted into the receiving part. For example, the first side connection port 34 can be inserted into the first receiving part 24, and the second side connection port 36 can be inserted into the second receiving part 26. Then, the side connection port is connected to the side port. For example, the first side connection port 34 can be connected to the first side port 84, and the second side connection port 36 can be connected to the second side port 86, by manipulating the control lever 114 of the holder apparatus 110. Further, a tube can be connected to at least one of the first end port 80 or the second end port 82 using a connector (not shown).

In the above embodiment, the case where the filter 70 includes four ports is shown. The number of ports that the filter includes is not limited to four but may be two, three, or five or more. The filter can include at least one end port and at least one side port. In this case, the holder apparatus 10 can include at least one support part, at least one receiving part, at least one end connection port, and at least one side connection port. In this case, the holder apparatus 110 can include at least one support part, at least one receiving part, and at least one side connection port.

In the embodiment described above, the case where the filter 70 is a particulate filter for filtration of a dialysate is shown, but the holder apparatus 10, 110 may be used to hold and connect a filter for a desired application. For example, the filter 70 may be a dialyzer for hemodialysis. In this case, the first end port 80 and the second end port 82 may be the inlet and the outlet connected to a blood circuit, and the first side port 84 and the second side port 86 may be the inlet and the outlet for the dialysate.

Described above is an explanation based on an exemplary embodiment. The embodiment is intended to be illustrative only and it will be understood by those skilled in the art that various design changes are possible and various modifications are possible and that such modifications are also within the scope of the present invention.

Several aspects of the present invention will be described.

The first aspect of the present invention relates to a holder apparatus including: a support part that supports a filter extending in an axial direction, regulates displacement of the filter in the axial direction, and permits rotation of the filter around the axial direction; a receiving part that receives a side port extending from a side of the filter in a radial direction and regulates displacement of the side port in the radial direction, when the filter supported by the support part is rotated in a predetermined direction around the axial direction; and a side connection port that is displaced in a direction approaching the side port placed in the receiving part and that is adapted to be connected to the side port. According to the first aspect, the displacement of the filter in the radial direction can be regulated by rotating the filter and inserting the side port into the receiving part. As a result, the filter is prevented from being misaligned even when a force is applied to the filter in the radial direction to connect the side connection port to the side port. This can improve the workability of mounting the filter.

The second aspect of the present invention relates to the holder apparatus according to the first aspect, wherein the receiving part permits displacement of the side port in the predetermined direction toward a connection position facing the side connection port and regulates further displacement of the side port from the connection position in the predetermined direction. According to the second aspect, the side port can be guided to the proper connection position by regulating the range of rotation of the side port inserted in the receiving part. As a result, the workability of mounting the filter can be improved.

The third aspect of the present invention relates to the holder apparatus according to the second aspect, wherein the receiving part has a notch extending toward the connection position in the predetermined direction. According to the third aspect, providing the notch in the receiving part makes it possible to guide the side port to the proper connection position by rotating the filter. As a result, the workability of mounting the filter can be improved.

The fourth aspect of the present invention relates to the holder apparatus according to the third aspect, wherein the notch is shaped to have an aperture width in the axial direction that is smaller toward the connection position. According to the fourth aspect, the side port inserted in the receiving part can be guided to the proper connection position by configuring the aperture width to be smaller toward the connection position. As a result, the workability of mounting the filter can be improved.

The fifth aspect of the present invention relates to the holder apparatus according to any one of the first through fourth aspects, further including: a rotation suppression part that suppresses rotation of the filter in a direction opposite to the predetermined direction while the side port is placed at a connection position facing the side connection port. According to the fifth aspect, the misalignment of the side port from the proper connection position can be suppressed so that the workability of mounting the filter can be improved.

The sixth aspect of the present invention relates to the holder apparatus according to the fifth aspect, wherein the rotation suppression part has a protrusion that engages with a rib provided on the side of the filter. According to the sixth aspect, the protrusion that engages with the rib of the filter suppresses the misalignment of the side port from the proper connection position so that the workability of mounting the filter can be improved.

The seventh aspect of the present invention relates to the holder apparatus according to the fifth aspect, wherein the rotation suppression part has a biasing means that applies a force that pulls the side port placed in the receiving part in the radial direction. According to the seventh aspect, the misalignment of the side port from the proper connection position can be suppressed by pulling the side port in the radial direction so that the workability of mounting the filter can be improved.

The eighth aspect of the present invention relates to the holder apparatus according to any one of first through seventh aspects, further including: a switching part that switches between a connected state in which the side connection port is biased toward the side port and a released state in which the side connection port is separated from the side port. According to the eighth aspect, the switching part can switch between the connected state and the released state so that the workability of connecting the side connection port to the side port can be improved.

The ninth aspect of the present invention relates to the holder apparatus according to the eighth aspect, further including: a detection part that detects that the side port is placed at a connection position facing the side connection port, wherein the switching part is configured to switch from the released state to the connected state in response to detection by the detection part. According to the ninth aspect, it is possible to switch to the connected state automatically by detecting that the side port is placed at the proper connection position so that the workability of connecting the side connection port to the side port can be improved.

The tenth aspect of the present invention relates to the holder apparatus according to the eighth aspect, further including: a detection part that detects that the side port is placed at a connection position facing the side connection port; and a switching inhibition part that permits state switching by the switching part when the detection part detects placement and inhibits state switching by the switching part when the detection part does not detect placement. According to the tenth aspect, the connection work is permitted only when the side port is placed at the proper connection position by providing the switching inhibition part so that connection failure due to misalignment of the side port can be prevented.

The eleventh aspect of the present invention relates to the holder apparatus according to any one of first through tenth aspects, further including: a main body on which the support part is mounted, wherein the receiving part is mounted on the main body to be swingable with respect to the main body. According to the eleventh aspect, even when the side port is misaligned from the design position due to tolerance in manufacturing, etc., a swinging motion of the receiving part with respect to the main body allows the side port at the misaligned position to be inserted into the receiving part.

The twelfth aspect of the present invention relates to the holder apparatus according to any one of the first through eleventh aspects, wherein the support part supports an end port extending from an end of the filter in the axial direction. According to the twelfth aspect, the filter can be supported to be rotatable around the central axis by supporting the end port of the filter.

The thirteenth aspect of the present invention relates to the holder apparatus according to the twelfth aspect, further including: an end connection port connected to the end port in the support part. According to the thirteenth aspect, the end connection port can be connected to the end port supported by the support part so that the workability of mounting the filter can be improved.

The fourteenth aspect of the present invention relates to the holder apparatus according to any one of the first through eleventh aspects, wherein the support part includes a first support part that supports a first end port extending from a first end of the filter in the axial direction and a second support part that supports a second end port extending in the axial direction from a second end of the filter opposite to the first end. According to the fourteenth aspect, the filter can be supported to be rotatable around the central axis by supporting both ends of the filter.

The fifteenth aspect of the present invention relates to the holder apparatus according to the fourteenth aspect, further including: a first end connection port that is displaced in the axial direction and connected to the first end port in the first support part, and a second end connection port that is displaced in the axial direction and connected to the second end port in the second support part. According to the fifteenth aspect, the first end connection port and the second end connection port can be connected to the first end port and the second end port so that the workability of mounting the filter can be improved.

The sixteenth aspect of the present invention relates to a mounting method including: causing a filter extending in an axial direction to be supported by a support part of a holder apparatus; rotating the filter supported by the support part in a predetermined direction around the axial direction and inserting a side port extending from a side of the filter in a radial direction into a receiving part of the holder apparatus; and connecting a side connection port of the holder apparatus to the side port placed in the receiving part. According to the sixteenth aspect, the filter can be fixed to the holder apparatus, and the side connection port can be connected to the side port in three steps including: insertion, rotation, and connection so that the workability of mounting the filter is improved.

### INDUSTRIAL APPLICABILITY

According to the present invention, the workability of mounting a filter on a holder apparatus can be improved.

### REFERENCE SIGNS LIST

10 ... holder apparatus, 12 ... main body, 14 ... first arm, 16 ... second arm, 20 ... first support part, 22 ... second support part, 24 ... first receiving part, 26 ... second receiving part, 30 ... first end connection port, 32 ... second end connection port, 34 ... first side connection port, 36 ... second side connection port, 38 ... first control lever, 40 ... second control lever, 42 ... switching part, 52 ... rotation suppression, 54 ... protrusion, 62 ... detection part, 70 ... filter, 74 ... first end, 76 ... second end, 78 ... side, 80 ... first end port, 82 ... second end port, 84 ... first side port, 86 ... second side port, 88 ... rib

## Claims

1. A holder apparatus comprising:
a support part that supports a filter extending in an axial direction, regulates displacement of the filter in the axial direction, and permits rotation of the filter around the axial direction;
a receiving part that receives a side port extending from a side of the filter in a radial direction and regulates displacement of the side port in the radial direction, when the filter supported by the support part is rotated in a predetermined direction around the axial direction; and
a side connection port that is displaced in a direction approaching the side port placed in the receiving part and that is adapted to be connected to the side port.

2. The holder apparatus according to Claim 1,
wherein the receiving part permits displacement of the side port in the predetermined direction toward a connection position facing the side connection port and regulates further displacement of the side port from the connection position in the predetermined direction.

3. The holder apparatus according to Claim 2,
wherein the receiving part has a notch extending toward the connection position in the predetermined direction.

4. The holder apparatus according to Claim 3,
wherein the notch is shaped to have an aperture width in the axial direction that is smaller toward the connection position.

5. The holder apparatus according to Claim 1, further comprising:
a rotation suppression part that suppresses rotation of the filter in a direction opposite to the predetermined direction while the side port is placed at a connection position facing the side connection port.

6. The holder apparatus according to Claim 5,
wherein the rotation suppression part has a protrusion that engages with a rib provided on the side of the filter.

7. The holder apparatus according to Claim 5,
wherein the rotation suppression part has a biasing means that applies a force that pulls the side port placed in the receiving part in the radial direction.

8. The holder apparatus according to any one of Claims 1 through 7, further comprising:
a switching part that switches between a connected state in which the side connection port is biased toward the side port and a released state in which the side connection port is separated from the side port.

9. The holder apparatus according to Claim 8, further comprising:
a detection part that detects that the side port is placed at a connection position facing the side connection port,
wherein the switching part is configured to switch from the released state to the connected state in response to detection by the detection part.

10. The holder apparatus according to Claim 8, further comprising:
a detection part that detects that the side port is placed at a connection position facing the side connection port; and
a switching inhibition part that permits state switching by the switching part when the detection part detects placement and inhibits state switching by the switching part when the detection part does not detect placement.

11. The holder apparatus according to any one of Claims 1 through 10, further comprising:
a main body on which the support part is mounted,
wherein the receiving part is mounted on the main body to be swingable with respect to the main body.

12. The holder apparatus according to any one of Claims 1 through 11,
wherein the support part supports an end port extending from an end of the filter in the axial direction.

13. The holder apparatus according to Claim 12, further comprising:
an end connection port connected to the end port in the support part.

14. The holder apparatus according to any one of Claims 1 through 11,
wherein the support part includes a first support part that supports a first end port extending from a first end of the filter in the axial direction and a second support part that supports a second end port extending in the axial direction from a second end of the filter opposite to the first end.

15. The holder apparatus according to Claim 14, further comprising:
a first end connection port that is displaced in the axial direction and connected to the first end port in the first support part, and
a second end connection port that is displaced in the axial direction and connected to the second end port in the second support part.

16. A mounting method comprising:
causing a filter extending in an axial direction to be supported by a support part of a holder apparatus;
rotating the filter supported by the support part in a predetermined direction around the axial direction and inserting a side port extending from a side of the filter in a radial direction into a receiving part of the holder apparatus; and
connecting a side connection port of the holder apparatus to the side port placed in the receiving part.
